# EUROPÄISCHE PATENTSCHRIFT

(11) **EP 3 745 896 B1**
(45) Veröffentlichungstag und Bekanntmachung des Hinweises auf die Patenterteilung: **09.03.2022**
(21) Anmeldenummer: 19705699.7
(22) Anmeldetag: 10.01.2019
(51) Int. Cl.: A43B 7/14, A43B 17/02, A43D 1/02, A43B 17/00, A43B 17/14, A43B 7/28

(54) **VORRICHTUNG ZUM ANFORMEN VON SCHUHEINLAGEN**
APPARATUS FOR MOULDING INSOLES
DISPOSITIF DESTINÉ À FORMER DES SEMELLES ORTHOPÉDIQUES

(30) Priorität: 30.01.2018 AT 500802018
(43) Veröffentlichungstag der Anmeldung: 09.12.2020
(73) Patentinhaber: Wintersteiger AG, 4910 Ried im Innkreis (AT)
(72) Erfinder: LACHINGER, Andreas, 4563 Micheldorf (AT); HOLZBERGER, Alexander, 4563 Micheldorf (AT)
(74) Vertreter: Hübscher & Partner Patentanwälte GmbH
(86) Internationale Anmeldenummer: PCT/AT2019/050003
(87) Internationale Veröffentlichungsnummer: WO 2019/148217

(56) Entgegenhaltungen:
- EP-A1- 0 310 531
- DE-A1-102016 100 644
- FR-A1- 2 682 028

## Beschreibung

### Technisches Gebiet

Die Erfindung bezieht sich auf eine Vorrichtung zum Anformen von Schuheinlagen an einen Fuß mit einem auf einem Träger angeordneten, einen Fußaufnahmebereich bildenden Formkissen.

### Stand der Technik

Es sind Vorrichtungen zum Anformen von Schuheinlagen bekannt

(US 20160288440 A1, FR 2 682 028 A1 ), die ein auf einem Träger angeordnetes, von einem flexiblen Schutzüberzug eingehülltes Kissen zeigen. Das Kissen weist dabei einen Schichtenaufbau auf, wobei die dem Träger zugewandte Schicht aus einem Schaumstoff und die darauf lösbar angeordnete, einen Fußaufnahmebereich bildendende zweite Schicht aus einem gelartigen Material besteht. Beim Anformen wird eine vorgewärmte, thermoformbare Schuheinlage im Fußaufnahmebereich angeordnet und anschließend ein Fuß auf die Einlage aufgesetzt. Damit ein Anformen der Schuheinlage erfolgen kann, muss der Fuß mit seinem Fußgewölbe zumindest teilweise in die Gelschicht eindringen, was nur unter Aufbringung größere Kräfte möglich ist, sodass ein Anformen in der Regel nur mithilfe einer im Stehen aufbringbaren Gewichtskraft erfolgen kann. Dazu kommt, dass die Gewichtskraft nicht gleichmäßig auf den ganzen Fuß aufgebracht werden kann, sodass die Gelschicht insbesondere im Vorfußbereich über eine angesetzte Grifflasche an den Fuß angedrückt werden muss, bis die Einlage im angeformten Zustand abgekühlt und damit formstabil ist. Folglich bedarf es beim Anformen einer Hilfestellung und es kann zufolge der instabilen, nachgiebigen Gelschicht kein sicherer Stand des Benützers sichergestellt werden.

### Darstellung der Erfindung

Die Erfindung betrifft eine Vorrichtung zum Anformen von Schuheinlagen, wie er im unabhängigen Anspruch 1 angegeben ist, und ein Verfahren zum Anformen von Schuheinlagen mit einer solchen Vorrichtung nach Anspruch 6. Bevorzugte Ausführungsformen der Erfindung sind in den abhängigen Ansprüchen offenbart.

Der Erfindung liegt somit die Aufgabe zugrunde, eine Vorrichtung der eingangs geschilderten Art so auszugestalten, dass ein Anformen von Schuheinlagen ohne Hilfestellung und unabhängig von einer Steh- oder Sitzposition des Benützers erfolgen kann.

Die Erfindung löst die gestellte Aufgabe dadurch, dass das Formkissen eine biegeelastische Membran zur Auflage der Schuheinlage aufweist, die über eine Fußauflagepunktfixierung mit einer Auflagefläche des Trägers verbunden ist und dass zwischen der Membran und der Auflagefläche ein Füllbereich für ein Fluid zum Anformen der Schuheinlage an den Fuß vorgesehen ist. Zufolge der erfindungsgemäßen Merkmale kann der Fuß des Benützers beim Anformen in einer Ruhestellung verbleiben, weil die Schuheinlage unabhängig von der vom Benützer aufgebrachten Gewichtskraft durch Füllen des Füllbereichs mit einem Fluid über die Membran an den Fuß angeformt wird. Der Anformdruck kann dabei über die Füllmenge bzw. den Fluiddruck innerhalb des Füllbereiches reguliert werden, wobei sich vorteilhafte Anformbedingungen ergeben, wenn der Anformdruck so gewählt wird, dass er einerseits eine Verformung der Schuheinlage ermöglicht und andererseits die Gewichtskraft des Fußes des Benützers in sitzender Position nicht oder nur geringfügig übersteigt. Um dabei dennoch eine zuverlässige Positionierung der Schuheinlage und des Fußes zueinander zu ermöglichen und insbesondere ein Wegrutschen des Fußes zu vermeiden, ist erfindungsgemäß die biegeelastische Membran über eine Fußauflagepunktfixierung mit einer Auflagefläche des Trägers verbunden, sodass die Membran im Bereich der Fußauflagepunktfixierung nicht durch das Fluid angehoben werden kann, sodass ein definierter Auflagebereich zwischen der Schuheinlage und dem Fuß gegeben ist. Vorteilhafterweise befindet sich die Fußauflagepunktfixierung im Fersenabschnitt des Fußaufnahmebereiches, weil die Ferse des Benützers eine besonders einfache Positionierung erlaubt. Es ist aber beispielsweise auch denkbar, die Fußauflagepunktfixierung im Ballenabschnitt anzuordnen.

Um die Ausrichtung der anzuformenden Schuheinlage und des Fußes des Benützers zueinander zu erleichtern, wird vorgeschlagen, dass im Fußaufnahmebereich des Formkissens eine Ausrichtungshilfe vorgesehen ist, die einen biegeelastischen Randbereich aufweist. Während die Ausrichtungshilfe bereits teilweise an eine gewöhnliche Fußform angepasst sein kann, um die Position der Schuheinlage und des Fußes vorzugeben, ermöglicht der biegeelastische Randbereich der Ausrichtungshilfe eine zuverlässige Anformung abhängig von der tatsächlichen Form und der Größe des Fußes des Benützers.

Um besonders einfache Konstruktionsbedingungen zu schaffen, kann die Ausrichtungshilfe über die Fußauflagepunktfixierung mit der Auflagefläche des Trägers verbunden sein. Wird die Ausrichtungshilfe oberhalb der Membran angeordnet, so muss der Füllbereich für das Fluid genauso wie die Membran nur einmal, nämlich durch die Fußauflagepunktfixierung, durchsetzt werden. Die Fußauflagepunktfixierung und die Membran müssen in diesem Fall selbstverständlich fluiddicht abschließen.

Damit auch im Bereich der vorgeformten Ausrichtungshilfe eine vollständige Anformung der Schuheinlage an die Fußform des Benützers erreicht werden kann, kann die Ausrichtungshilfe innerhalb des Füllbereiches angeordnet sein. Dies bedeutet, dass die Ausrichtungshilfe beim Füllen des Füllbereiches mit einem Fluid in ihrer relativen Lage zur Auflagefläche bzw. zum Träger bleibt, während sich die Membran zur Anformung der Schuheinlage von der Ausrichtungshilfe abhebt und an die Fußform des Benützers anschmiegt. Beim einem Entleeren des Fluides aus dem Füllbereich ermöglicht dieses Merkmal auch eine Rückstellung des Randbereiches der Ausrichtungshilfe in eine Ausgangslage, was insbesondere bei einer häufigen Verwendung der erfindungsgemäßen Vorrichtung einen zuverlässigen, langfristigen Betrieb sicherstellt.

Je nach Größe des Fußes des Benützers kann sich das Problem ergeben, dass auch eine an sich unerwünschte Anformung der Schuheinlage im Zehenbereich erfolgt. Um dies zu verhindern, wird vorgeschlagen, dass die Vorrichtung einen Schieber zur Anschlagsbegrenzung der Membran im ballenseitigen Fußaufnahmebereich aufweist. Dadurch ergibt sich im Zehenbereich ein zusätzlicher stabiler Auflagepunkt für den Fuß des Benützers, was die Positionierung des Fußes weiter begünstigt.

Die Erfindung bezieht sich auch auf ein Verfahren zum Anformen von Schuheinlagen mit einer Vorrichtung nach einem der vorangegangenen Ansprüche, wobei die Schuheinlage bei weitgehend leerem Füllbereich auf der Membran angeordnet und nach dem Aufsetzen des Fußes auf die Schuheinlage der Füllbereich mit einem Fluid gefüllt wird, und dass nach dem Anformen der Schuheinlage der Füllbereich zur Freigabe des Fußes und der Schuheinlage entleert wird. Im weitgehend entleerten Zustand des Füllbereiches tritt somit die Ausrichtungshilfe durch die Membran hervor und erleichtert die Positionierung der Schuheinlage sowie des Fußes. Besonders vorteilhafte Bedingungen ergeben sich in Verbindung mit thermoformbaren Schuheinlagen, wenn das verwendete Fluid gekühlt wird, weil es dadurch zu einem rascheren Abkühlen der vorgewärmten Schuheinlage und damit zu einer frühzeitigeren Formstabilität kommt. Bei dem eingesetzten Fluid kann es sich beispielsweise um Luft oder Wasser handeln, wobei ersteres eine einfache Handhabung der erfindungsgemäßen Vorrichtung erlaubt.

### Kurze Beschreibung der Erfindung

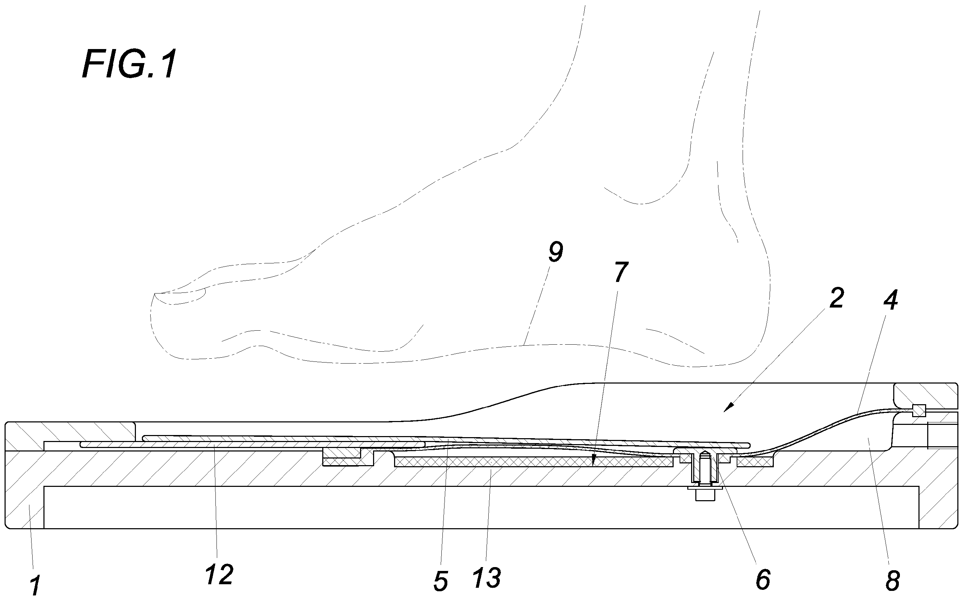

In der Zeichnung ist der Erfindungsgegenstand beispielsweise dargestellt. Es zeigen
- Fig. 1: eine erfindungsgemäße Vorrichtung in einer ersten Ausführungsform in einem schematischen Schnitt bei entleertem Füllbereich,

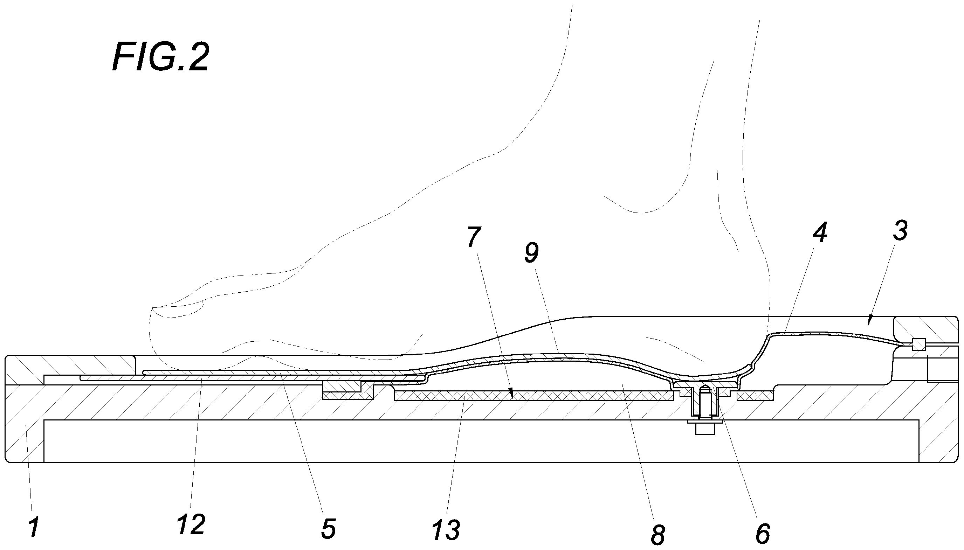

- Fig. 2: eine der Fig. 1 entsprechende Darstellung bei gefülltem Füllbereich und aufgesetztem Fuß eines Anwenders,

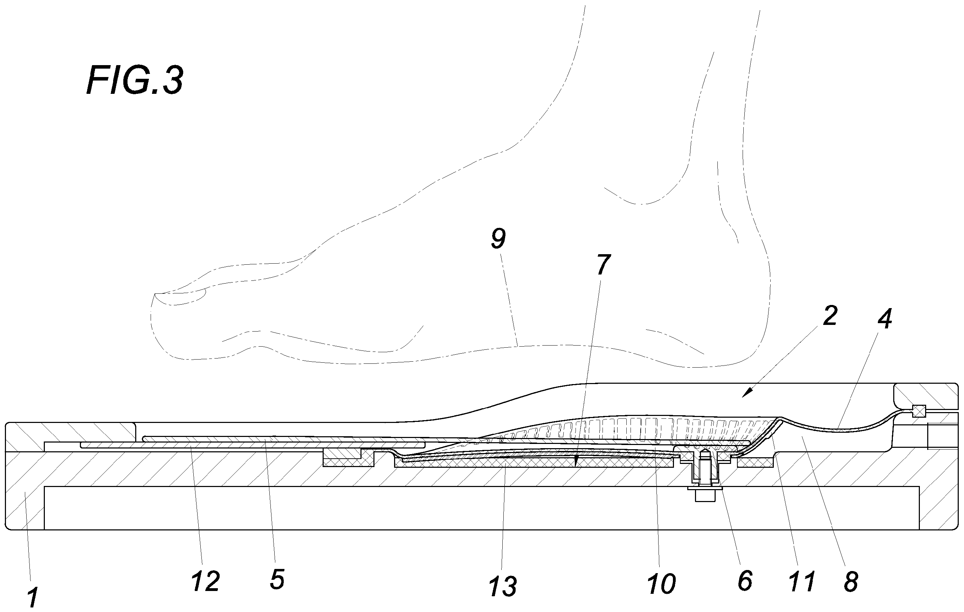

- Fig. 3: eine erfindungsgemäße Vorrichtung in einer zweiten Ausführungsform in einer der Fig. 1 entsprechenden Darstellung und

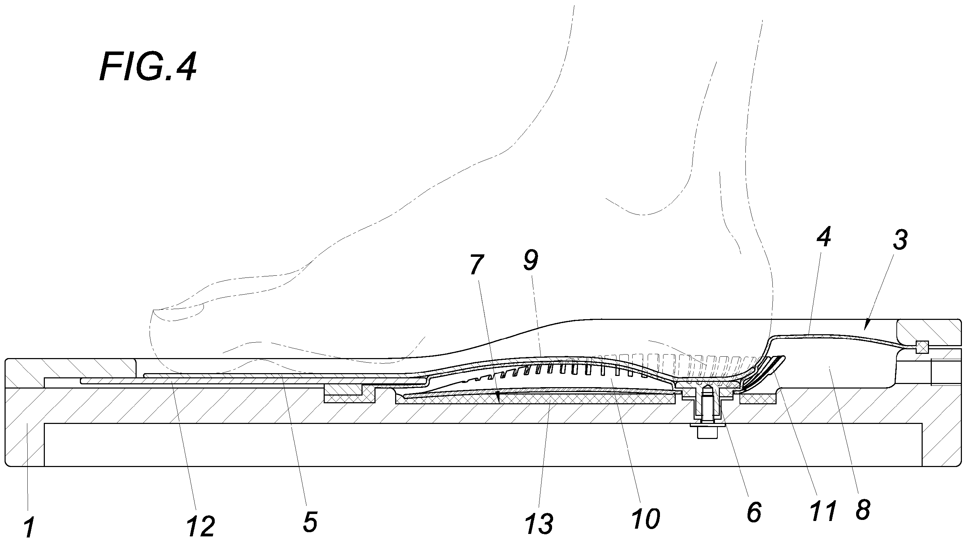

- Fig. 4: eine der Fig. 3 entsprechende Darstellung bei gefülltem Füllbereich und aufgesetztem Fuß eines Anwenders.

### Wege zur Ausführung der Erfindung

Eine erfindungsgemäße Vorrichtung weist ein auf einem Träger 1 angeordnetes, einen Fußaufnahmebereich 2 bildendes Formkissen 3 auf, das eine biegeelastische Membran 4 zur Auflage einer Schuheinlage 5 aufweist. Die Membran 4 ist zudem über eine Fußauflagepunktfixierung 6 mit einer Auflagefläche 7 des Trägers 1 verbunden. Zwischen der Membran 4 und der Auflagefläche 7 des Trägers 1 ist ein Füllbereich 8 für ein Fluid zum Anformen der Schuheinlage 5 an den Fuß 9 eines Benützers vorgesehen. Der Füllbereich kann beispielsweise dadurch gebildet werden, dass die Membran eine füllbare Blase bildet oder dass die Membran fluiddicht mit dem Träger abschließt.

Bei einem Anformvorgang wird zuerst die Schuheinlage 5 im Fußaufnahmebereich 2 der Membran 4 angeordnet und sodann der Fuß 9 des Benützers auf die Schuheinlage 5 aufgesetzt. In weiterer Folge wird die Membran 4 mit Druck beaufschlagt, in dem der zunächst leere Füllbereich 8 mit einem Fluid gefüllt wird. Zufolge der Expansion der Membran 4 drückt diese die formbare Schuheinlage 5 formgebend an den Fuß 9 an. Nachdem die Schuheinlage 5 auf diese Weise an den Fuß 9 des Benützers angepasst worden ist, wird der Füllbereich 8 wieder entleert, sodass der Fuß 9 sowie die angeformte Schuheinlage 5 aus dem Fußaufnahmebereich 2 entfernt werden können.

Um die Ausrichtung der Schuheinlage 5 und des Fußes 9 zueinander zu vereinfachen, kann gemäß einer Ausführungsform der erfindungsgemäßen Vorrichtung eine Ausrichtungshilfe 10 im Fußaufnahmebereich 2 des Formkissens 3 vorgesehen sein. Um trotz einer konturgebenden Ausgestaltung der Ausrichtungshilfe 10 einen Anformvorgang nicht zu behindern, kann die Ausrichtungshilfe 10 einen biegeelastischen Randbereich 11 aufweisen, sodass sich die Schuheinlage 5 zufolge der Druckbeaufschlagung der Membran 4 an den Fuß 9 des Benützers anschmiegen kann.

Um einfache Konstruktionsbedingungen zu schaffen, kann es gemäß einer Ausführungsform vorgesehen sein, dass die Ausrichtungshilfe 10 über die Fußauflagepunktfixierung 6 mit der Auflagefläche 7 des Trägers 1 verbunden ist. Beispielsweise kann die Fußauflagepunktfixierung 6 mit dem Träger 1 verschraubt sein.

Damit sich die Membran 4 beim Anformen vollständig an den Fuß 9 des Benützers anschmiegen kann, kann die Ausrichtungshilfe 10 innerhalb des Füllbereiches 8 angeordnet sein. Dadurch ergibt sich der weitere Vorteil, dass sich beim Entleeren des Füllbereiches 8 die Membran 4 so an die Ausrichtungshilfe 10 anschmiegen kann, dass die Positionierung von Schuheinlage 5 und Fuß 9 noch einfacher vorgenommen werden kann.

Um eine unerwünschte Anformung der Schuheinlage 5 bei unterschiedlichen Schuhgrößen beispielsweise an den Zehenbereich zu vermeiden, kann die Vorrichtung zudem einen Schieber 12 zur Anschlagsbegrenzung der Membran 4 im ballenseitigen Fußaufnahmebereich 2 aufweisen. Dabei kann der Schieber 12 zweckmäßigerweise vor einem Anformvorgang in Trägerlängsrichtung so verschoben werden, dass die Länge des Fußaufnahmebereiches 2 eingestellt und folglich der anzuformende Abschnitt der Schuheinlage 5 festgelegt werden kann.

Werden thermoformbare Schuheinlagen 5 eingesetzt, kann erfindungsgemäß vorgesehen sein, dass das in den Füllbereich 8 einströmende Fluid gekühlt wird, sodass die thermoformbaren Schuheinlagen 5 schneller in einem formstabilen Zustand aushärten können, nachdem sie an den Fuß 9 angeformt worden sind.

Zudem kann gemäß einer Ausführungsform der erfindungsgemäßen Vorrichtung ein nachgiebiges Pufferelement 13, beispielsweise aus einem Schaumstoff, vorgesehen sein, das zwischen der Ausrichtungshilfe 10 und der Auflagefläche 7 des Trägers 1 angeordnet ist, um eine angenehmere, taktile Wahrnehmung des Benützers beim Positioniervorgang des Fußes 9 bei weitgehend leerem Füllbereich 8 zu ermöglichen.

## Patentansprüche

1. Vorrichtung zum Anformen von Schuheinlagen (5) an einen Fuß (9) mit einem auf einem Träger (1) angeordneten, einen Fußaufnahmebereich (2) bildenden Formkissen (3), das eine biegeelastische Membran (4) zur Auflage der Schuheinlage (5) aufweist, wobei die Membran (4) über eine Fußauflagepunktfixierung (6) mit einer Auflagefläche (7) des Trägers (1) verbunden ist und dass zwischen der Membran (4) und der Auflagefläche (7) ein Füllbereich (8) für ein Fluid zum Anformen der Schuheinlage (5) an den Fuß (9) vorgesehen ist.

2. Vorrichtung nach Anspruch 1, **dadurch gekennzeichnet, dass** im Fußaufnahmebereich (2) des Formkissens (3) eine Ausrichtungshilfe (10) vorgesehen ist, die einen biegeelastischen Randbereich (11) aufweist.

3. Vorrichtung nach Anspruch 2, **dadurch gekennzeichnet, dass** die Ausrichtungshilfe (10) über die Fußauflagepunktfixierung (6) mit der Auflagefläche (7) des Trägers (1) verbunden ist.

4. Vorrichtung nach einem der Ansprüche 2 bis 3, **dadurch gekennzeichnet, dass** die Ausrichtungshilfe (10) innerhalb des Füllbereiches (8) angeordnet ist.

5. Vorrichtung nach einem der Ansprüche 1 bis 4, **dadurch gekennzeichnet, dass** die Vorrichtung einen Schieber (12) zur Anschlagsbegrenzung der Membran (4) im ballenseitigen Fußaufnahmebereich (2) aufweist.

6. Verfahren zum Anformen von Schuheinlagen (5) mit einer Vorrichtung nach einem der vorangegangenen Ansprüche, wobei die Schuheinlage (5) bei weitgehend leerem Füllbereich (8) auf der Membran (4) angeordnet und nach dem Aufsetzen des Fußes (9) auf die Schuheinlage (5) der Füllbereich (8) mit einem Fluid gefüllt wird, und dass nach dem Anformen der Schuheinlage (5) der Füllbereich (8) zur Freigabe des Fußes (9) und der Schuheinlage (5) entleert wird.

## Claims

1. Apparatus for moulding insoles (5) on a foot (9), having a moulding pad (3) which is arranged on a support (1), forms a foot-accommodating region (2) and has a flexible membrane (4) for supporting the insole (5), wherein the membrane (4) is connected to a bearing surface (7) of the support (1) via a foot-bearing-point fixing means (6), and that a filling region (8) for a fluid for moulding the insole (5) on the foot (9) is provided between the membrane (4) and the bearing surface (7).

2. Apparatus as claimed in claim 1, **characterised in that** an alignment aid (10) is provided in the foot-accommodating region (2) of the moulding pad (3) and has a flexible edge region (11).

3. Apparatus as claimed in claim 2, **characterised in that** the alignment aid (10) is connected to the bearing surface (7) of the support (1) via the foot-bearing-point fixing means (6).

4. Apparatus as claimed in any one of claims 2 to 3, **characterised in that** the alignment aid (10) is arranged within the filling region (8).

5. Apparatus as claimed in any one of claims 1 to 4, **characterised in that** the apparatus has a slide (12) for limiting the stop of the membrane (4) in the foot-accommodating region (2) on the ball side.

6. Method of moulding insoles (5) with an apparatus as claimed in any one of the preceding claims, wherein the insole (5) is arranged on the membrane (4) when the filling region (8) is largely empty and after placing the foot (9) on the insole (5) the filling region (8) is filled with a fluid, and that after moulding the insole (5) the filling region (8) is emptied in order to release the foot (9) and the insole (5).

## Revendications

1. Dispositif pour le formage d'une semelle intérieure (5) sur un pied (9) avec un coussin de formage (3) formant une zone de réception de pied (2), disposé sur un support (1), qui présente une membrane élastique en flexion (4) pour l'application de la semelle intérieure (5), dans lequel la membrane (4) est reliée par une fixation de point d'application du pied (6) avec une surface d'application (7) du support (1) et qu'entre la membrane (4) et la surface d'application (7) est prévue une zone de remplissage (8) pour un fluide destiné au formage de la semelle intérieure (5) sur le pied (9).

2. Dispositif selon la revendication 1, **caractérisé en ce que** dans la zone de réception de pied (2) du coussin de formage (3), il est prévu un accessoire d'orientation (10) qui présente une zone de bordure élastique en flexion (11).

3. Dispositif selon la revendication 2, **caractérisé en ce que** l'accessoire d'orientation (10) est relié par la fixation de point d'application du pied (6) avec la surface d'application (7) du support (1).

4. Dispositif selon une des revendications 2 à 3, **caractérisé en ce que** l'accessoire d'orientation (10) est disposé à l'intérieur de la zone de remplissage (8).

5. Dispositif selon une des revendications 1 à 4, **caractérisé en ce que** ce dispositif présente une glissière (12) pour la limitation de la butée de la membrane (4) dans la zone de réception du pied (2) du côté éminence.

6. Procédé pour le formage de semelles intérieures (5) avec un dispositif selon une des revendications précédentes, selon lequel la semelle intérieure (5) est disposée sur la membrane (4) lorsque la zone de remplissage (8) est sensiblement vide et la zone de remplissage (8) est remplie avec un fluide après le positionnement du pied (9) sur la semelle intérieure (5), et qu'après le formage de la semelle intérieure (5), la zone de remplissage (8) est vidée pour libérer le pied (9) et la semelle intérieure (5).
